# EUROPÄISCHE PATENTSCHRIFT

(11) **EP 2 904 983 B1**
(45) Veröffentlichungstag und Bekanntmachung des Hinweises auf die Patenterteilung: **15.05.2019**
(21) Anmeldenummer: 14153938.7
(22) Anmeldetag: 05.02.2014
(51) Int. Cl.: A61B 18/00, A61B 18/04, A61B 18/14

(54) **Elektrochirurgisches Instrument mit einem Drehrad als Betätigungselement und mit einer Brems- oder Arretiereinrichtung**
Electrosurgical instrument with an actuating wheel and with a brake or locking device
Instrument électrochirurgical avec un actionneur rotatif et avec un dispositif de freinage or de blocage

(43) Veröffentlichungstag der Anmeldung: 12.08.2015
(73) Patentinhaber: Erbe Elektromedizin GmbH, 72072 Tübingen (DE)
(72) Erfinder: Kirstgen, Udo, 72108 Rottenburg (DE); Buntrock, Volker, 72762 Reutlingen (DE); Alberstetter, Tobias, 72116 Mössingen (DE); Rombach, Thorsten, 72810 Gomaringen (DE); Ahlburg, Elmar, 72818 Trochtelfingen (DE)
(74) Vertreter: Bohnenberger, Johannes

(56) Entgegenhaltungen:
- US-A- 5 258 006
- US-A- 5 295 614
- US-A1- 2007 010 801
- US-B1- 6 190 360

## Beschreibung

Die Erfindung bezieht sich auf ein elektrochirurgisches Instrument, insbesondere für die Argonplasma-Koagulation, mit den Merkmalen des Oberbegriffs des Anspruchs 1. Ein solches Instrument ist beispielsweise aus US 2009/0125023 A1 bekannt. Die Erfindung betrifft ferner ein Gerät mit einem solchen Instrument.

US 6 190 360 B1 beschreibt ein elektrochirurgisches Instrument mit einem Schaft, einem Betätigungsmechanismus in Form eines Drehrads und mit einer Arretiereinrichtung.

Elektrochirurgische Instrumente der eingangs genannten Art werden zum Schneiden von Gewebe oder zum Koagulieren mit hochfrequentem Wechselstrom eingesetzt. Die Argonplasma-Koagulation ist eine spezielle Anwendungsform der Elektrochirurgie, bei der HF-Strom kontaktlos über ionisiertes Argongas übertragen wird.

Die Wirkung des Energieeintrages erfolgt bei dem eingangs genannten bekannten Instrument dadurch, dass die exponierte Länge der Elektrode geändert wird. Dazu ist ein axial beweglicher Schaft vorgesehen, der die Elektrode isolierend umgibt und entlang der Elektrode verschoben werden kann, um diese je nach Bedarf zu exponieren.

Eine zentrale Anforderung an derartige Instrumente ist die Möglichkeit der Ein-Hand-Bedienung. Dabei soll sich die Position des Instruments im OP-Feld möglichst nicht ändern. Dies bedeutet, dass die Griffhaltung bei der Bedienung des Instruments möglichst beibehalten werden soll, auch wenn der Schaft verschoben wird. Bei dem gattungsgemäßen Instrument wird dies durch ein Drehrad erreicht, das zentral im Handgriff des Instrumentes angeordnet ist und mit dem Zeigefinger betätigt werden kann. Das Drehrad treibt den Schaft an, der dadurch entlang der Elektrode axial verschoben werden kann.

Eine weitere Anforderung an elektrochirurgische Instrumente ergibt sich aus der Verwendung im Zusammenhang mit einem Trokar, der zum Einführen der Elektrode genutzt wird. Dabei kann es beim Verschieben des Instruments im Trokar zu Reibungskräften zwischen dem Schaft und dem Trokar kommen, die den Schaft zurückhalten und die Elektrode ungewollt freisetzen.

Der Erfindung liegt die Aufgabe zugrunde, das eingangs genannte elektrochirurgische Instrument dahingehend zu verbessern, dass die Verletzungsgefahr bei der Verwendung des Instruments im Zusammenhang mit einem Trokar oder auch bei der Präparation verringert wird, ohne dabei die Handhabung des Gerätes zu beeinträchtigen. Der Erfindung liegt ferner die Aufgabe zugrunde, ein Gerät mit einem solchen Instrument anzugeben.

Erfindungsgemäß wird diese Aufgabe durch ein elektrochirurgisches Instrument mit dem Merkmal des Anspruchs 1 bzw. durch ein Gerät mit den Merkmalen des Anspruchs 13 gelöst.

Insbesondere wird die Aufgabe durch ein elektrochirurgisches Instrument, insbesondere für die Argonplasma-Koagulation gelöst, das einen Handgriff sowie eine mit dem Handgriff verbundene Elektrode aufweist. Ein Schaft umgibt die Elektrode, der im Handgriff gehalten ist. Das Instrument umfasst einen Betätigungsmechanismus, der wenigstens ein am Handgriff angeordnetes Drehrad aufweist. Der Schaft ist relativ zur Elektrode axial beweglich und durch Betätigen des Drehrades mit einer Schubkraft beaufschlagbar. Der Handgriff weist eine Bremseinrichtung zur permanenten Ausübung einer Bremskraft auf den Schaft auf. Der Betätigungsmechanismus bildet ein Übersetzungsgetriebe, das mit dem Schaft zur Übertragung der Schubkraft verbunden ist.

Die Erfindung hat verschiede Vorteile:
Die Sicherheit des Instruments gegen ungewolltes Exponieren der Elektrode beispielsweise bei der Verwendung zusammen mit einem Trokar wird durch die Bremseinrichtung verbessert. Die von der Bremseinrichtung auf den Schaft ausgeübte Bremskraft verhindert, dass dieser bei einer Anwendung, beispielsweise beim Einführen durch einen Trokar in proximaler Richtung verschoben wird. Die Bremskraft führt also zu einer Selbsthemmung des Schaftes, die diesen gegen ungewolltes Verschieben sichert.

Zur Änderung der exponierten Länge der Elektrode ist der Schaft durch Betätigen eines Drehrades mit einer Bestätigungskraft beaufschlagbar. Diese Kraft wird üblicherweise durch den Finger des Anwenders aufgebracht. Um den Anwender bei der Bedienung des Instrumentes nicht zu behindern, sollte der Widerstand des Drehrades in einem als ergonomisch angenehm empfundenen Bereich bleiben. Dazu weist das erfindungsgemäße Instrument ein Übersetzungsgetriebe auf, das durch den Betätigungsmechanismus gebildet ist und mit dem Schaft zur Übertragung der Schubkraft verbunden ist. Das Übersetzungsgetriebe gleicht die von der Bremseinrichtung aufgebrachte Bremskraft aus, so dass das Drehrad bzw. allgemein der Betätigungsmechanismus leicht zu bedienen ist.

Es versteht sich, dass das Drehrad in zwei Richtungen (Uhrzeigersinn / Gegenuhrzeigersinn) drehbar ist, sodass der Schaft in distaler Richtung und in proximaler Richtung bewegt werden kann.

Zusammengefasst erhöht die Erfindung die Sicherheit des Instruments, weil der Schaft gegen ungewolltes Verschieben durch die Bremseinrichtung gesichert ist. Gleichzeitig wird eine leichtgängige Bedienung des Instruments beibehalten, da der Betätigungsmechanismus ein Übersetzungsgetriebe bildet, das die vom Anwender aufgebrachte Fingerkraft in die auf den Schaft wirkende Schubkraft umwandelt. Das Übersetzungsgetriebe wirkt dabei wie eine Hebelanordnung, die die Schubkraft im Vergleich zur Fingerkraft erhöht.

Vorzugsweise umfasst das Drehrad ein Antriebsrad und wenigstens ein drehfest mit dem Antriebrad verbundenes Abtriebsrad, das mit dem Schaft zur Übertragung der Schubkraft verbunden ist. Der Durchmesser des Abtriebsrades ist kleiner als der Durchmesser des Antriebsrades. Damit wird auf einfache Weise die für eine leichtgängige Betätigung des Schaftes erforderliche Übersetzung erreicht. Ein weiterer Vorteil dieser Ausführungsform ist die kostengünstige und sichere Konstruktion, die diese Ausführungsform bietet.

Der Betätigungsmechanismus kann einen in Schubrichtung axial beweglichen Schlitten umfassen, der einerseits mit dem Schaft und andererseits mit dem Übersetzungsgetriebe verbunden ist. Damit wird eine robuste und einfache Konstruktion geschaffen, die eine sichere Übertragung der vom Anwender aufgebrachten Antriebskraft auf den Schaft bewirkt.

Der Schlitten kann wenigstens eine erste Zahnstange aufweisen, die parallel zur Schubrichtung angeordnet ist und mit dem Abtriebsrad kämmt. Diese Ausführung ermöglicht eine einfache und sichere Umwandlung der Drehbewegung des Drehrades in eine lineare Bewegung des Schaftes.

Für eine verbesserte Kraftübertragung kann der Schlitten eine zweite Zahnstange parallel zur ersten Zahnstange aufweisen, wobei das Antriebsrad zwischen den beiden Zahnstangen angeordnet und mit einem weiteren Abtriebsrad drehfest verbunden ist. Das weitere Abtriebsrad ist mit der zweiten Zahnstange verzahnt.

Vorzugsweise weist der Handgriff eine Halteplatte mit einer Linearführung auf, in der der Schlitten axial beweglich angeordnet ist. Die Linearführung weist wenigstens einen Durchbruch, insbesondere zwei parallele Durchbrüche, für den Schlitten auf. Die Halteplatte ermöglicht einen kompakten Aufbau, der einen geringen Bauraum für die Lagerung des Schlittens erfordert.

Der Bremsmechanismus kann ein Klemmelement, insbesondere einen Klemmring aufweisen, wobei das Klemmelement im Handgriff gehalten ist und den Schaft mit der Bremskraft beaufschlagt. Das Klemmelement bildet ein passives Bremsmittel, das einen einfachen und kostengünstigen Aufbau des Instrumentes ermöglicht.

Bei einer bevorzugten Ausführungsform erweist der Betätigungsmechanismus eine Arretiereinrichtung auf, mit der der Schaft in wenigstens einer Position, insbesondere in einer vollständig ausgefahrenen Position, fixierbar ist. Die Arretiereinrichtung ist besonders für Trokare geeignet, die einen besonders großen Widerstand beim Einführen des Instrumentes bewirken, wie beispielsweise wiederverwendbare Trokare mit Ventilklappe. Die Arretiereinrichtung dient dazu, den Schaft zusätzlich zum Bremsmechanismus zu fixieren, so dass größere Axialkräfte vom Schaft übertragen werden können, ohne dass dieser relativ zur Elektrode verschoben wird.

Dabei kann die Arretiereinrichtung wenigstens ein erstes Rastmittel umfassen, das am Schlitten angeordnet ist. Ein zweites Rastmittel ist am Handgriff, insbesondere an der Haltplatte angeordnet, das mit dem ersten Rastmittel zur Fixierung des Schaftes verbindbar ist. Die beiden Rastmittel haben den Vorteil, dass diese einfach herstellbar sind, beispielsweise durch ein Spritzgussverfahren, und gleichzeitig eine sichere Fixierung des Schaftes ermöglichen.

Bei einer besonders bevorzugten Ausführungsform sind die Elektrode und der Schaft relativ zum Handgriff jeweils um ihre Längsachse drehbar angeordnet. Die Elektrode ist durch eine Schiebehülse geführt, die den Schaft und die Elektrode drehfest und axial beweglich verbindet.

Diese Ausführungsform ist für nicht-rotationssymmetrische Elektroden, wie beispielsweise Spatelektroden geeignet. Damit kann auf einfache Weise die Elektrode in Umfangsrichtung ausgerichtet werden. Diese Ausführungsform hat den Vorteil, dass die Drehung der Elektrode auch dann möglich ist, wenn sich das Instrument im Trokar befindet. Die Drehbewegung wird bei dieser Ausführungsform durch den Schaft eingeleitet, der über die Schiebehülse mit der Elektrode drehfest verbunden ist. Die Schiebehülse hat zudem die Funktion, die Relativbeweglichkeit zwischen dem Schaft und der Elektrode herzustellen. Dazu bildet die Schiebehülse eine drehfeste und axial bewegliche Verbindung zwischen dem Schaft und der Elektrode. Da der Schaft aus dem Handgriff hervorragt, sind keine zusätzlichen Bauteile erforderlich, um die Elektrode zu drehen. Der Anwender greift einfach den Schaft und dreht diesen zusammen mit der Elektrode.

Dabei kann die Schiebhülse zumindest abschnittsweise eine Profilierung auf dem Innenumfang aufweisen, die mit der zumindest abschnittsweise entsprechend profilierten Elektrode zur Übertragung eines Drehmoments formschlüssig in Eingriff ist. Diese Ausführung ist kostengünstig und sicher, da eine entsprechend profilierte Schiebehülse einfach herstellbar ist und durch den Formschluss eine sichere Momentenübertragung erreicht wird.

Ein kostengünstiger und einfacher Aufbau wird vorzugsweise dadurch erreicht, dass die Schiebehülse und der Schlitten drehbeweglich und in axialer Richtung der Schiebehülse zur Übertragung der Schubkraft fest verbunden sind. Der Schlitten weist einen Haltering auf, der die Schiebehülse zumindest teilumfänglich umgibt.

Die Erfindung wird nachfolgend mit weiteren Einzelheiten und mit Bezug auf die beigefügten schematischen Figuren näher erläutert. Diese zeigen:
- Fig. 1: eine perspektivische Ansicht des Instrumentes nach einem erfindungsgemäßen Ausführungsbeispiel, bei dem das Gehäuse teilweise entfernt ist;
- Fig. 2: einen Querschnitt des Instrumentes gemäß Fig. 1, wobei das Drehrad weggelassen ist, und
- Fig. 3: einen Längsschnitt des Instruments gemäß Fig. 1 entlang der Mittelachse.

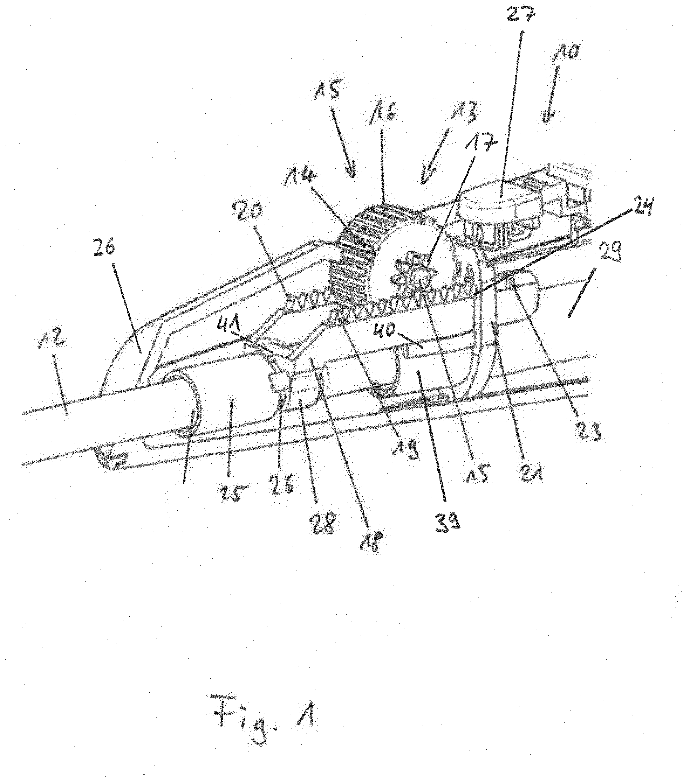

Das Ausführungsbeispiel gemäß den Figuren 1 bis 3 zeigt ein elektrochirurgisches Instrument, das für die Argonplasma-Koagulation einsetzbar ist. Die Erfindung ist nicht auf Instrumente für die Argonplasma-Koagulation eingeschränkt, sondern ist allgemein für Instrumente im Bereich der Elektrochirurgie einsetzbar, bei denen eine Elektrode durch Verschieben eines Schaftes aktiviert und/oder geregelt wird.

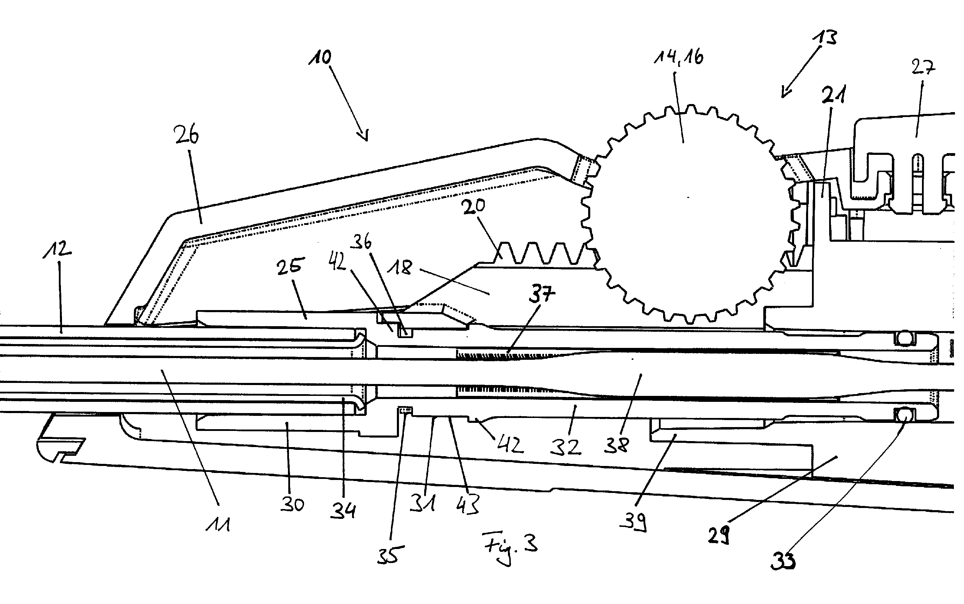

Bei der Elektrode 11 des Ausführungsbeispiels kann es sich bspw. um eine Hohlelektrode handeln, die einen Kanal für die Gaszuführung aufweist (APC-Elektrode). Andere Elektroden sind möglich. Die Elektrode 11 ist in einem Handgriff 10 gelagert. Der Handgriff 10 weist Anschlüsse oder Zuleitungen für die Elektrode auf, die die Stromversorgung und ggf. die Gaszufuhr der Elektrode 11 ermöglichen. Außerdem sind ein oder mehrere Betätigungsmittel, beispielsweise Druckknöpfe 27 am Handgriff vorgesehen. Die Elektrode 11 ist in einem verschiebbaren Schaft 12 angeordnet, der über den Handgriff in distaler Richtung vorsteht und im Gehäuse 26 des Handgriffs 10 gehalten ist (s. Fig. 3). Der Schaft 12 ist aus einem isolierenden Material hergestellt und umgibt die Elektrode 11 zumindest im Bereich außerhalb des Handgriffes 10.

Der Schaft 12 ist relativ zur Elektrode 11 verschiebbar, so dass durch eine Axialbewegung des Schaftes 12 die Elektrode 11 am distalen Ende (nicht dargestellt) in verschiedenen Positionen, insbesondere stufenlos, exponiert werden kann. Dadurch kann der Bereich der Elektrode, der bei der Verwendung des Instruments mit Gewebe in Kontakt kommen kann, geregelt werden. In seiner Endposition in distaler Richtung übergreift der, koaxial zur Elektrode angeordnete Schaft 12, die Elektrode 11 in ihrer gesamten Länge.

Das Instrument weist eine Bremseinrichtung auf, die den Schaft 12 permanent mit einer Bremskraft beaufschlagt und als Verschiebesicherung wirkt. Die Bremskraft oder auch Selbsthemmung des Schaftes 12 wirkt der Widerstandskraft beim Einführen des Instrumentes in einen Trokar entgegen und verhindert, dass der Schaft 12 ungewollt in proximaler Richtung verschoben wird. Der Vorteil der Verschiebesicherung des Schaftes 12 kommt auch in anderen Situationen zum Tragen, bspw. bei der Präparation.

Konkret weist die Bremseinrichtung ein kraftschlüssig wirkendes Klemmelement, beispielsweise in der Form eines Klemmrings 33 auf (Figur 3). Der Klemmring 33 kann ein O-Ring sein. Andere passive Bremsmittel, die der Widerstandskraft im Trokar entgegenwirken, sind möglich. Der Klemmring 33 ist zumindest indirekt mit dem Schaft 12 verbunden und überträgt die in den Schaft 12 eingeleiteten Axialkräfte in den Handgriff 10, konkret in das Gehäuse 26 des Handgriffs 10. Dazu ist der Schaft 12 mit einer Schiebehülse 25 verbunden. Die Schiebehülse 25 und der Schaft 12 sind koaxial angeordnet. Die Schiebehülse 25 kann als eine axiale Verlängerung des Schaftes 12 in den Handgriff 10 hinein verstanden werden. Am distalen Ende der Schiebhülse 25 ist der Klemmring 33 in einer passenden Nut angeordnet derart, dass der Klemmring 33 über den Außenumfang der Schiebehülse 25 vorsteht. Der Klemmring 33 ist im Handgriff 10 abgestützt und erzeugt eine Bremskraft, die einer Kraft die in Längsrichtung auf den Schaft 12 wirkt, beispielsweise der Widerstandskraft im Trokar entgegen wirkt.

Konkret ist die Schiebehülse 25 koaxial in einer Innenhülse 29 angeordnet, die fest mit dem Gehäuse 26, insbesondere durch eine Halteplatte 21, verbunden ist. Der Klemmring 33 drückt gegen den Innenumfang der Innenhülse 29 und erzeugt so eine axial wirkende Bremskraft. Die Innenhülse 29 bildet zugleich die axiale Führung der Schiebehülse 25.

Der Klemmring 33 bzw. allgemein die Bremseinrichtung kann an einer anderen Stelle der Schiebehülse 25 angeordnet sein. Es ist auch möglich, mehr als einen Klemmring 33, beispielsweise zwei Klemmringe zu verwenden.

Um zu vermeiden, dass die Bremseinrichtung die Handhabung des Instrumentes erschwert, bildet der Betätigungsmechanismus 13 ein Übersetzungsgetriebe 15, das mit dem Schaft 12 zur Übertragung der Schubkraft verbunden ist.

Der Betätigungsmechanismus 13 weist ein Drehrad 14 auf, das zumindest teilweise aus dem Gehäuse 26 des Handgriffes 10 vorsteht, so dass ein Teilumfang des Drehrades 14 für die Betätigung durch einen Finger zugänglich ist. Die Drehbewegung des Drehrades 14 bewirkt die axiale Verschiebung des Schaftes 12. Durch Betätigung des Drehrades 14 im Uhrzeigersinn bzw. gegen den Uhrzeigersinn kann der Schaft in distaler Richtung vorgeschoben bzw. in proximaler Richtung zurückgezogen werden. Mit anderen Worten kann der Schaft 12 hin und her bewegt werden.

Die Funktion des Übersetzungsgetriebes 15 besteht darin, das in das Drehrad 14 eingeleitete Drehmoment so zu wandeln, dass der Schaft mit einer erhöhten Schubkraft beaufschlagt wird. Das Übersetzungsgetriebe 15 ist so angepasst, dass die Fingerkraft zur Betätigung des Drehrades 14 kleiner als die Selbsthemmung des Schaftes 12 ist.

Das Übersetzungsgetriebe 15 umfasst das Drehrad 14, das wiederum ein Antriebsrad 16 und wenigstens ein drehfest mit dem Antriebsrat 16 verbundenes Abtriebsrad 17 aufweist (Figur 1). Das Abtriebsrad 17 ist als Zahnrad ausgebildet, das koaxial mit dem Antriebsrad 16 verbunden ist. Das Antriebsrad 16 kann zur sicheren Bewegung Haltemittel beispielweise in Form einer Riffelung auf dem Außenumfang aufweisen. Dadurch ist das präzise Bewegen des Antriebsrads 16 mittels eines Fingers gewährleistet. Das Drehrad 14 kann als Stufenrad ausgebildet sein, wobei das Antriebsrad 16 und das Abtriebsrad 17 einstückig bzw. integral ausgebildet sind. Alternativ können das Antriebsrad 16 und das Abtriebsrad 17 mechanisch miteinander verbunden sein.

Wie in Fig. 1 gut zu erkennen, ist der Durchmesser des Antriebsrades 16 größer als der Durchmesser des Abtriebsrades 17. Konkret ist der Außendurchmesser des Antriebrades 16 um das ca. 2,8-fache größer als der Durchmesser des Abtriebsrades 17. Somit beträgt das Hebelverhältnis ca. 1:2,8. Die erforderliche Fingerkraft ist damit um das 2,8-fache geringer als die Selbsthemmung des Schaftes 12. Das Hebelverhältnis kann im Bereich von 1:2,6-3,0 liegen, insbesondere im Bereich 1:2,7-2,9.

Ein weiterer Vorteil des Übersetzungsgetriebes besteht darin, dass der Verschiebeweg bzw. das zurückgelegte Bogenmaß am Außendurchmesser des Antriebsrades 16 ebenfalls das 2,8-fache bzw. ein Vielfaches des Verschiebewegs des Schaftes 12 beträgt. Damit kann eine besonders genaue Einstellung der Schaftposition und damit des Exponierungsgrades der Elektrode 11 erreicht werden.

Im vorliegenden Ausführungsbeispiel beträgt der Außendurchmesser des Antriebsrades 16 ca. 12,5 mm. Die für die Selbsthemmung des verschiebbaren Schaftes 12 erforderliche Bremskraft bzw. Klemmkraft beträgt ca. 4 Newton.

Die Umwandlung des vom Drehrad 14 aufgebrachten Drehmomentes in eine translatorische Schubbewegung des Schaftes 12 wird durch einen Schlitten 18 erreicht, der in proximaler und distaler Richtung axial beweglich ist. Der Schlitten 18 bildet die Verbindung zwischen dem Schaft 12 und dem Übersetzungsgetriebe 15. Dazu weist der Schlitten 18 eine erste Zahnstange 19 auf, die parallel zur Schubrichtung des Schaftes 12 angeordnet ist. Die erste Zahnstange 19 ist mit dem Abtriebsrad 17 verzahnt. Andere Konstruktionen zur Umwandlung der Drehbewegung in eine translatorische Bewegung sind möglich. Im Beispiel gemäß Fig. 1 ist die Zahnstange 19 außenliegend angeordnet. Alternativ kann eine innenliegende Zahnstange vorgesehen sein, die an der Innenseite eines Längsschlitzes ausgebildet ist, der sich parallel zur Mittelachse der Elektrode 11 erstreckt. Das Abtriebsrad 17 ist dann in dem Längsschlitz angeordnet.

Wie Fig. 1 zu entnehmen, weist der Schlitten 18 eine zweite Zahnstange 20 auf, die parallel zur ersten Zahnstange 19 angeordnet ist. Das Antriebsrad 16 ist zwischen den beiden Zahnstangen 19 und 20 angeordnet und mit einem weiteren Abtriebsrad 17 drehfest verbunden. Das weitere Abtriebsrad 17 (nicht dargestellt) kämmt mit der zweiten Zahnstange 20. Der symmetrische Aufbau des Betätigungsmechanismuses 13 führt zu einer gleichmäßigen Krafteinleitung sowie zu einer verbesserten Sicherheit des Instruments.

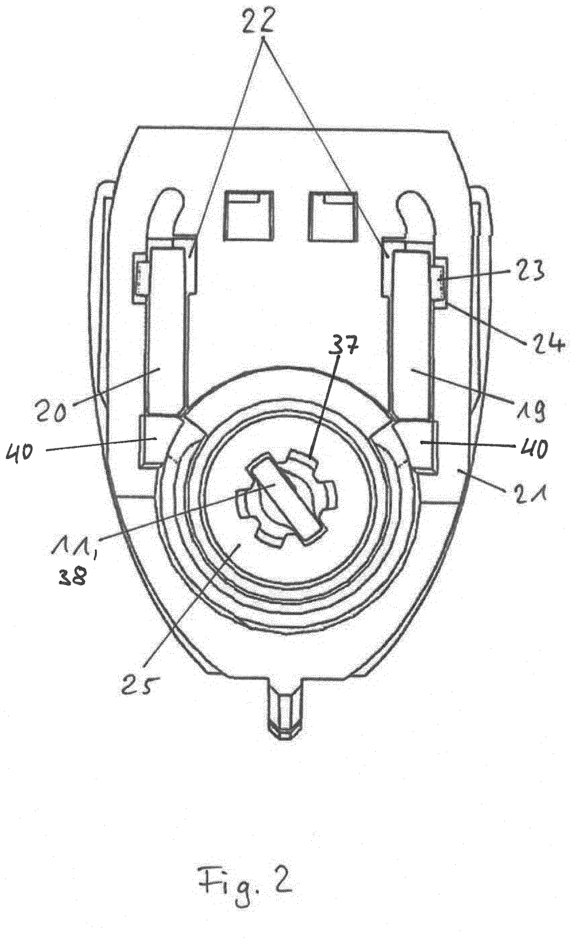

Die beiden Zahnstangen 19, 20 bilden zwei Arme, die sich parallel zur Längsachse der Elektrode 11 erstrecken. Die beiden Zahnstangen 19, 20 sind in einer Linearführung angeordnet, die durch die Halteplatte 21 gebildet ist. Die Halteplatte 21 sitzt fest im Gehäuse 26 und weist zwei parallele Durchbrüche 22 für den Schlitten 18 auf (Figur 2). Durch die beiden Durchbrüche 22 sind die Zahnstangen 19, 20 geführt, so dass eine sichere translatorische Bewegung des Schlittens 18 möglich ist. Das Drehrad 14 ist zwischen den beiden Zahnstangen 19, 20 vor der Halteplatte 21 angeordnet, wodurch ein kompakter Aufbau des Handgriffs 10 erreicht wird.

Eine weitere Verbesserung der Sicherheit wird durch eine Arretiereinrichtung am Schlitten 18 erreicht. Die Arretiereinrichtung dient dazu, den Schaft 12 in einer vorgegebenen Position, insbesondere in der vollständig ausgefahrenen Position zu fixieren, so dass die Elektrode 11 möglichst vollständig vom Schaft 12 überdeckt ist. Damit kann das Instrument zusammen mit einem Trokar verwendet werden, dessen Reibungs- bzw. Klemmkraft größer als die Bremskraft des Handgriffes 10 ist.

Im Unterschied zu der Arretiereinrichung, die den Schlitten 18 in einer bestimmten Position festlegt, wirkt die Bremseinrichtung in jeder Position des Schlittens 18, so dass eine stufenlose Verstellung des Schaftes 12 möglich ist.

Konkret weist die Arretiereinrichtung ein erstes Rastmittel 23 auf, das am proximalen Ende jeweils der ersten und zweiten Zahnstange 19, 20 angeordnet ist. Das erste Rastmittel 23 wirkt im Arretierzustand mit einem zweiten Rastmittel 24 zusammen, das am Handgriff 10 ausgebildet ist. Konkret ist das zweite Rastmittel 24 an der Halteplatte 21 in der Form einer Rastausnehmung ausgebildet. Das erste Rastmittel 23 kann eine entsprechend ausgebildete Rastnase sein, die seitlich an den beiden Zahnstangen 19, 20 angeordnet ist.

Die Arretiereinrichtung verbessert die Gesamtsicherheit des Instruments gemäß Figuren 1-3. Es ist auch möglich, die Arretiereinrichtung unabhängig vom Übersetzungsgetriebe und der Bremseinrichtung zu verwenden, beispielsweise dann, wenn das Instrument ausschließlich mit Trokaren mit sehr hoher Widerstandskraft, wie bspw. bei wiederverwendbaren Trokaren mit Ventilklappe, verwendet werden soll.

Ein weiterer Vorteil des Instruments gemäß Figuren 1-3 besteht darin, dass die Elektrode 11 in Umfangsrichtung ausgerichtet werden kann, selbst dann, wenn der Schaft 12 zumindest teilweise in einem Trokar eingeführt ist. Dazu sind die Elektrode 11 und der Schaft 12 jeweils relativ zum Handgriff 10 um ihre Längsachse drehbar angeordnet. Mit anderen Worten können die Elektrode 11 und der Schaft 12 zusammen verdreht werden. Dazu ist die Schiebehülse 25 vorgesehen, durch die die Elektrode 11 geführt ist. Die Schiebehülse 25 verbindet den Schaft 12 und die Elektrode 11. Dabei handelt es sich um eine drehfeste und axial bewegliche Verbindung. Die Schiebehülse 25 ermöglicht also die Übertragung eines Drehmoments vom Schaft 12 auf die Elektrode 11. Gleichzeitig kann die Schiebehülse 25 und somit der mit dieser koaxial bzw. fluchtend verbundene Schaft 12 relativ zur Elektrode 11 in axialer Richtung verschoben werden.

Diese Doppelfunktion (Drehmomentübertragung und axiale Verschiebbarkeit) wird dadurch erreicht, dass die Schiebehülse 25 zumindest abschnittsweise eine Profilierung 37 auf dem Innenumfang aufweist. Die Elektrode 11 ist im Bereich der Profilierung 37 entsprechend profiliert und steht zur Übertragung des Drehmoments formschlüssig mit der Schiebehülse 25 in Eingriff. Die formschlüssige Verbindung ist so gestaltet, dass die Schiebehülse 25 entlang der Elektrode 11 sowohl in distaler als auch in proximaler Richtung bewegt werden kann.

Konkret weist die Schiebehülse 25 wenigstens drei Abschnitte auf, nämlich einen distalen Hülsenabschnitt 30, einen mittleren Hülsenabschnitt 31 und einen proximalen Hülsenabschnitt 32. Die Profilierung 37 ist im Bereich des proximalen Hülsenabschnitts 32 ausgebildet. Die Bremseinrichtung, konkret der Klemmring 33 ist am proximalen Ende des proximalen Hülsenabschnittes 32 angeordnet. Die Profilierung 37 erstreckt sich über eine Länge, die in etwa der Länge der beiden Zahnstangen 19, 20 entspricht. Damit wird erreicht, dass die formschlüssige Verbindung zwischen der Elektrode 11 und der Profilierung 37 in jeder Relativlage der Schiebehülse 25 beibehalten wird, so dass die Drehfunktion unabhängig von der jeweiligen Lage des Schaftes 12 gegeben ist.

Wie in Fig. 2 dargestellt, ist die Profilierung 37 nach Art eines Keilwellenprofils ausgebildet. Dies erhöht die Montagefreundlichkeit, da die entsprechend profilierte Elektrode 11 in die Schiebehülse im Wesentlichen unabhängig von deren Drehposition eingeschoben werden kann. Die Elektrode 11 weist einen Profilabschnitt 38 mit einem rechteckigen Querschnitt auf, wie in Fig. 2 dargestellt. Das proximale und distale Ende des Profilabschnitts 38 der Elektrode 11 verjüngt sich jeweils, wie in Fig. 3 gezeigt. Distal und proximal vom Profilabschnitt 38 weist die Elektrode in herkömmlicher Weise einen im Wesentlichen kreisförmigen Querschnitt auf. Am distalen Ende der Elektrode kann der Querschnitt in einen nicht-rotationssymmetrischen Querschnitt übergehen. Die Elektrode kann bspw. eine Spatelektrode sein.

Der mittlere Hülsenabschnitt 31 weist in distaler und proximaler Richtung jeweils eine Schulter 42 auf. Zwischen den beiden Schultern 42 ist ein Haltebereich 43 ausgebildet, der drehbeweglich mit dem Schlitten 18 verbunden ist. Der Haltebereich 43 bildet eine Ausnehmung zwischen den beiden Schultern 42. In dieser Ausnehmung ist ein Haltering 28 des Schlittens 18 angeordnet. Der Haltering 28 ist teilweise geöffnet und umschließt die Schiebehülse nur teilumfänglich, so dass der Haltering 28 zur Montage einfach auf die Schiebehülse 25 aufgeclipst werden kann. Der Haltering 28 schlägt an den beiden Schultern 42 an, so dass Axialkräfte bzw. die Schubkraft in proximaler und distaler Richtung zum Bewegen des Schaftes 12 übertragen werden können. Als weitere Sicherheit weist der mittlere Hülsenabschnitt 31 eine Ringnut 35 auf, in der ein Mitnehmer 36 des Halterings 28 angeordnet ist. Der Mitnehmer 36 und die Ringnut 35 sind relativ zueinander drehbeweglich, so dass die Hülse 25 im Haltering 28 frei drehbar ist. Der Mitnehmer 36 überträgt ebenfalls die Schubkraft in beiden axialen Richtungen.

Der Haltering 28 ist zwischen den beiden Zahnstangen 19, 20 an deren distalen Ende angeordnet. Konkret ist eine Traverse 41 vorgesehen, die die distalen Enden der beiden Zahnstangen 19, 20 verbindet, wie in Fig. 1 gezeigt. Die Traverse 41 wiederum ist mit dem Haltering 28 fest verbunden bzw. einstückig ausgebildet. Die Traverse 41 und der Haltering 28 können auch als eine Traverse mit zwei unten angeordneten Haltebacken angesehen werden, die die Schiebehülse 25 teilumfänglich umgeben.

Zwischen der Traverse 41 und der Halteplatte 21 ist ein ausreichender Abstand vorgesehen, so dass der Schlitten 18 am Drehrad 14 vorbei bewegt werden kann, ohne mit dem Drehrad 14 zu kollidieren.

Die Schiebehülse 25 umfasst ferner einen distalen Hülsenabschnitt 30. Der distale Hülsenabschnitt 30 ist drehfest mit dem Schaft 12 verbunden. Die Verbindung kann mechanisch erfolgen, beispielsweise durch eine Befestigungshülse 34, die im Schaft 12 angeordnet und am proximalen Ende des Schaftes 12 mit der Schiebehülse 25 vercrimpt ist. Andere Befestigungsmöglichkeiten sind denkbar. Der distale Hülsenabschnitt 30 bildet zusammen mit dem Gehäuse 26 einen axialen Anschlag, der die maximale Auszugsposition des Schaftes 12 bestimmt.

Zur Unterstützung der Linearführung des Schlittens 18 weist der Handgriff die vorstehend erwähnte Innenhülse 29 auf, die fest mit der Halteplatte 21 verbunden ist. Die Innenhülse 29 ist koaxial zur Elektrode 11 angeordnet und erstreckt sich distal und proximal von der Halteplatte 21, wie in den Figuren 1, 2 dargestellt. Auf der distalen Seite der Halteplatte 21 bildet die Innenhülse 29 einen Hülsenabschnitt 39 mit zwei Führungsstegen 40 , die sich parallel zur Mittelachse der Innenhülse 29 erstrecken. Die Führungsstege 40 bilden Auflageflächen für die beiden Zahnstangen 19, 20 und verbessern so die Stabilität der Linearführung.

Wie in Fig. 3 gezeigt, ist ein Teil der Umfangswand der Innenhülse 29 im Bereich des Drehrades 14 entfernt, um Raum für das Drehrad 14 zu schaffen, das bis auf das für die Fingerbetätigung erforderliche Umfangssegment im Gehäuse 26 angeordnet ist, ohne mit der Innenhülse 29 zu kollidieren. Dies trägt zu einem kompakten Aufbau des Handgriffs bei.

Die Drehfunktion des Handgriffes macht diesen für den Einsatz mit nicht-rotationssymmetrischen Elektroden geeignet, wie beispielsweise Spatelektroden, so dass der Handgriff nicht nur besonders sicher und kostengünstig ist, sondern auch auf unterschiedlichen Gebieten einsetzbar ist. Die Drehfunktion funktioniert auch mit anderen Handgriffen ohne Bremseinrichtung und Übersetzungsgetriebe. Das erfindungsgemäße Instrument wird zusätzlich auch im Zusammenhang mit einem Elektrochirurgiegerät, insbesondere für die Argonplasma-Koagulation offenbart und beansprucht.

### Bezugszeichenliste

- 10: Handgriff
- 11: Elektrode
- 12: Schaft
- 13: Betätigungsmechanismus
- 14: Drehrad
- 15: Übersetzungsgetriebe
- 16: Antriebsrad
- 17: Abtriebsrad
- 18: Schlitten
- 19: erste Zahnstange
- 20: zweite Zahnstange
- 21: Halteplatte
- 22: Durchbruch
- 23: erstes Rastmittel
- 24: zweites Rastmittel
- 25: Schiebehülse
- 26: Gehäuse
- 27: Druckknopf
- 28: Haltering
- 29: Innenhülse
- 30: distaler Hülsenabschnitt
- 31: mittlerer Hülsenabschnitt
- 32: proximaler Hülsenabschnitt
- 33: Klemmring
- 34: Befestigungshülse
- 35: Ringnut
- 36: Mitnehmer
- 37: Profilierung
- 38: Profilabschnitt
- 39: Hülsenabschnitt
- 40: Führungssteg
- 41: Traverse
- 42: Schulter
- 43: Haltebereich

## Patentansprüche

1. Elektrochirurgisches Instrument, insbesondere für die Argonplasma-Koagulation, mit
- einem Handgriff (10),
- einer mit dem Handgriff (10) verbundenen Elektrode (11),
- einem Schaft (12), der die Elektrode (11) umgibt und im Handgriff (10) gehalten ist, und
- einem Betätigungsmechanismus (13), der wenigstens ein am Handgriff (10) angeordnetes Drehrad (14) umfasst,
wobei der Schaft (12) relativ zur Elektrode (11) axial beweglich und durch Betätigen des Drehrades (14) mit einer Schubkraft beaufschlagbar ist,
**dadurch gekennzeichnet, dass**
der Handgriff (10) eine Bremseinrichtung zur permanenten Ausübung einer Bremskraft auf den Schaft (12) aufweist und der Betätigungsmechanismus (13) ein Übersetzungsgetriebe (15) bildet, das mit dem Schaft (12) zur Übertragung der Schubkraft verbunden ist.

2. Instrument nach Anspruch 1,
**dadurch gekennzeichnet, dass**
das Drehrad (14) ein Antriebsrad (16) und wenigstens ein drehfest mit dem Antriebsrad (16) verbundenes Abtriebsrad (17) umfasst, das mit dem Schaft (12) zur Übertragung der Schubkraft verbunden ist, wobei der Durchmesser des Abtriebsrades (17) kleiner als der Durchmesser des Antriebsrades (16) ist.

3. Instrument nach Anspruch 1 oder 2,
**dadurch gekennzeichnet, dass**
der Betätigungsmechanismus (13) einen in Schubrichtung axial beweglichen Schlitten (18) umfasst, der einerseits mit dem Schaft (12) und andererseits mit dem Übersetzungsgetriebe (15) verbunden ist.

4. Instrument nach Anspruch 3,
**dadurch gekennzeichnet, dass**
der Schlitten (18) wenigstens eine erste Zahnstange (19) aufweist, die parallel zur Schubrichtung angeordnet ist und mit dem Abtriebsrad (17) kämmt.

5. Instrument nach Anspruch 4,
**dadurch gekennzeichnet, dass**
der Schlitten (18) eine zweite Zahnstange (20) parallel zur ersten Zahnstange (19) aufweist, wobei das Antriebsrad (16) zwischen den beiden Zahnstangen (19, 20) angeordnet ist und mit einem weiteren Abtriebsrad (17) drehfest verbunden ist, das mit der zweiten Zahnstange (20) kämmt.

6. Instrument nach einem der Ansprüche 3 bis 5,
**dadurch gekennzeichnet, dass**
der Handgriff (10) eine Halteplatte (21) mit einer Linearführung aufweist, in der der Schlitten (18) axial beweglich angeordnet ist, wobei die Linearführung wenigstens einen Durchbruch (22), insbesondere zwei parallele Durchbrüche (22), für den Schlitten (18) aufweist.

7. Instrument nach einem der vorhergehenden Ansprüche,
**dadurch gekennzeichnet, dass**
die Bremseinrichtung ein Klemmelement, insbesondere einen Klemmring (33), aufweist, wobei das Klemmelement im Handgriff (10) gehalten ist und den Schaft (12) mit der Bremskraft beaufschlagt.

8. Instrument nach einem der vorhergehenden Ansprüche,
**dadurch gekennzeichnet, dass**
der Betätigungsmechanismus (13) eine Arretiereinrichtung aufweist, mit der der Schaft (12) in wenigstens einer Position, insbesondere in einer vollständig ausgefahrenen Position, fixierbar ist.

9. Instrument nach Anspruch 8,
**dadurch gekennzeichnet, dass**
die Arretiereinrichtung wenigstens ein erstes Rastmittel (23) umfasst, das am Schlitten (18) angeordnet ist, wobei ein zweites Rastmittel (24) am Handgriff (10), insbesondere an der Halteplatte (21), angeordnet ist, das mit dem ersten Rastmittel (23) zur Fixierung des Schaftes (12) verbindbar ist.

10. Instrument nach einem der vorhergehenden Ansprüche,
**dadurch gekennzeichnet, dass**
die Elektrode (11) und der Schaft (12) relativ zum Handgriff (10) jeweils um ihre Längsachse drehbar angeordnet sind, wobei die Elektrode (11) durch eine Schiebehülse (25) geführt ist, die den Schaft (12) und die Elektrode (11) drehfest und axial beweglich verbindet.

11. Instrument nach Anspruch 10,
**dadurch gekennzeichnet, dass**
die Schiebehülse (25) zumindest abschnittsweise eine Profilierung auf dem Innenumfang aufweist, die mit der zumindest abschnittsweise entsprechend profilierten Elektrode (11) zur Übertragung eines Drehmomentes formschlüssig in Eingriff ist.

12. Instrument nach Anspruch 10 oder 11,
**dadurch gekennzeichnet, dass**
die Schiebehülse (25) und der Schlitten (18) drehbeweglich und in axialer Richtung der Schiebehülse (25) zur Übertragung der Schubkraft fest verbunden sind, wobei der Schlitten (18) einen Haltering (28) aufweist, der die Schiebehülse (25) zumindest teilumfänglich umgibt.

13. Elektrochirurgiegerät, insbesondere für die Argonplasma-Koagulation, mit einem Instrument nach einem der vorhergehenden Ansprüche.

## Claims

1. Electrosurgical instrument, in particular for argon plasma coagulation, comprising
- a handle (10),
- an electrode (11) connected to the handle (10),
- a shaft (12) which surrounds the electrode (11) and is held in the handle (10), and
- an actuating mechanism (13) which comprises at least one rotary knob (14) arranged on the handle (10),
which shaft (12) is axially movable relative to the electrode (11) and can be subjected to a shearing force by actuation of the rotary knob (14),
**characterized in that**
the handle (10) has a brake device for permanently exerting a braking force on the shaft (12), and the actuating mechanism (13) forms a transmission gearing (15) which is connected to the shaft (12) in order to transmit the shearing force.

2. Instrument according to Claim 1,
**characterized in that**
the rotary knob (14) comprises a drive gear (16) and at least one driven gear (17) connected to the drive gear (16) for conjoint rotation therewith, said driven gear (17) being connected to the shaft (12) for transmitting the shearing force, the diameter of the driven gear (17) being smaller than the diameter of the drive gear (16).

3. Instrument according to Claim 1 or 2,
**characterized in that**
the actuating mechanism (13) comprises a carriage (18) which is axially movable in a shear direction and which is connected on the one hand to the shaft (12) and on the other hand to the transmission gearing (15).

4. Instrument according to Claim 3,
**characterized in that**
the carriage (18) has at least one first toothed rack (19), which is arranged parallel to the shear direction and meshes with the driven gear (17).

5. Instrument according to Claim 4,
**characterized in that**
the carriage (18) has a second toothed rack (20) parallel to the first toothed rack (19), the drive gear (16) being arranged between the two toothed racks (19, 20) and being connected to a further driven gear (17) for conjoint rotation therewith, which driven gear (17) meshes with the second toothed rack (20).

6. Instrument according to one of Claims 3 to 5,
**characterized in that**
the handle (10) has a retaining plate (21) with a linear guide in which the carriage (18) is arranged in an axially movable manner, the linear guide having at least one aperture (22), in particular two parallel apertures (22), for the carriage (18).

7. Instrument according to one of the preceding claims,
**characterized in that**
the brake device has a clamping element, in particular a clamping ring (33), which clamping element is held in the handle (10) and applies the braking force to the shaft (12).

8. Instrument according to one of the preceding claims,
**characterized in that**
the actuating mechanism (13) has a locking device with which the shaft (12) can be fixed in at least one position, in particular in a fully extended position.

9. Instrument according to Claim 8,
**characterized in that**
the locking device comprises at least one first latching means (23) which is arranged on the carriage (18), a second latching means (24) being arranged on the handle (10), in particular on the retaining plate (21), and being connectable to the first latching means (23) for fixing the shaft (12).

10. Instrument according to one of the preceding claims,
**characterized in that**
the electrode (11) and the shaft (12) are arranged to be rotatable relative to the handle (10) about their respective longitudinal axis, the electrode (11) being guided through a sliding sleeve (25) which connects the shaft (12) and the electrode (11) to each other in an axially movable manner and for conjoint rotation.

11. Instrument according to Claim 10,
**characterized in that**
the sliding sleeve (25) has, at least in part, a profiling on the inner circumference, which profiling is in form-fit engagement with the electrode (11), correspondingly profiled at least in part, in order to transmit a torque.

12. Instrument according to Claim 10 or 11,
**characterized in that**
the sliding sleeve (25) and the carriage (18) are movable in rotation and rigidly connected in the axial direction of the sliding sleeve (25) for transmitting the shearing force, the carriage (18) having a retaining ring (28) which surrounds the sliding sleeve (25) at least partly about the circumference.

13. Electrosurgical appliance, in particular for argon plasma coagulation, comprising an instrument according to one of the preceding claims.

## Revendications

1. Instrument électrochirurgical, notamment pour la coagulation au plasma d'argon, comprenant
- une poignée (10),
- une électrode (11) reliée à la poignée (10),
- une tige (12) qui entoure l'électrode (11) et qui est maintenue dans la poignée (10), et
- un mécanisme d'actionnement (13) qui comprend au moins une molette (14) disposée sur la poignée (10),
la tige (12) étant mobile axialement par rapport à l'électrode (11) et pouvant être sollicitée avec une force de poussée en actionnant la molette (14),
**caractérisé en ce que**
la poignée (10) possède un dispositif de freinage destiné à exercer en permanence une force de freinage sur la tige (12) et le mécanisme d'actionnement (13) forme un engrenage de démultiplication (15) qui est relié à la tige (12) en vue de la transmission de la force de poussée.

2. Instrument selon la revendication 1, **caractérisé en ce que** la molette (14) comporte une roue d'entraînement (16) et au moins une roue menée (17) reliée en rotation solidaire à la roue d'entraînement (16), laquelle est reliée à la tige (12) en vue de la transmission de la force de poussée, le diamètre de la roue menée (17) étant inférieur au diamètre de la roue d'entraînement (16).

3. Instrument selon la revendication 1 ou 2,
**caractérisé en ce que** le mécanisme d'actionnement (13) comporte un coulisseau (18) mobile axialement dans le sens de la poussée, lequel est relié d'un côté à la tige (12) et de l'autre côté à l'engrenage de démultiplication (15) .

4. Instrument selon la revendication 3, **caractérisé en ce que** le coulisseau (18) possède au moins une première crémaillère (19) qui est disposée parallèlement au sens de la poussée et s'engrène avec la roue menée (17).

5. Instrument selon la revendication 4, **caractérisé en ce que** le coulisseau (18) possède une deuxième crémaillère (20) parallèle à la première crémaillère (19), la roue d'entraînement (16) étant disposée entre les deux crémaillères (19, 20) et étant reliée en rotation solidaire à une roue menée (17) supplémentaire qui s'engrène avec la deuxième crémaillère (20).

6. Instrument selon l'une des revendications 3 à 5,
**caractérisé en ce que** la poignée (10) possède une plaque de maintien (21) dotée d'un guide linéaire dans lequel le coulisseau (18) est disposé avec mobilité axiale, le guide linéaire possédant au moins une percée (22), notamment deux percées (22) parallèles pour le coulisseau (18) .

7. Instrument selon l'une des revendications précédentes, **caractérisé en ce que** le dispositif de freinage possède un élément de serrage, notamment une bague de serrage (33), l'élément de serrage étant maintenu dans la poignée (10) et sollicitant la tige (12) avec la force de freinage.

8. Instrument selon l'une des revendications précédentes, **caractérisé en ce que** le mécanisme d'actionnement (13) possède un dispositif de blocage avec lequel la tige (12) peut être immobilisée dans au moins une position, notamment dans une position complètement sortie.

9. Instrument selon la revendication 8, **caractérisé en ce que** le dispositif de blocage comporte au moins un premier moyen d'encliquetage (23) qui est disposé sur le coulisseau (18), un deuxième moyen d'encliquetage (24) étant disposé sur la poignée (10), notamment au niveau de la plaque de maintien (21), lequel peut être relié au premier moyen d'encliquetage (23) en vue d'immobiliser la tige (12).

10. Instrument selon l'une des revendications précédentes, **caractérisé en ce que** l'électrode (11) et la tige (12) sont respectivement disposées rotatives autour de leur axe longitudinal par rapport à la poignée (10), l'électrode (11) étant guidée par une douille coulissante (25) qui relie la tige (12) et l'électrode (11) en rotation solidaire et avec mobilité axiale.

11. Instrument selon la revendication 10,
**caractérisé en ce que** la douille coulissante (25) possède au moins dans certaines portions un profilage sur le pourtour intérieur, lequel est en prise par complémentarité de formes avec l'électrode (11) profilée en conséquence au moins dans certaines portions en vue de transmettre un couple.

12. Instrument selon la revendication 10 ou 11,
**caractérisé en ce que** la douille coulissante (25) et le coulisseau (18) sont reliés avec mobilité en rotation et en position fixe dans la direction axiale de la douille coulissante (25) en vue de transmettre la force de poussée, le coulisseau (18) possédant une bague de maintien (28) qui entoure la douille coulissante (25) sur au moins une partie de son pourtour.

13. Appareil électrochirurgical, notamment pour la coagulation au plasma d'argon, comprenant un instrument selon l'une des revendications précédentes.
